Europäisches Patentamt

European Patent Office

Office européen des brevets

(19)

(11) **EP 1 290 976 A2**

(12) **EUROPÄISCHE PATENTANMELDUNG**

(43) Veröffentlichungstag:
   **12.03.2003   Patentblatt 2003/11**

(51) Int Cl.[7]: **A61B 5/0275**, A61B 5/029

(21) Anmeldenummer: **02017615.2**

(22) Anmeldetag: **05.08.2002**

(84) Benannte Vertragsstaaten:
   **AT BE BG CH CY CZ DE DK EE ES FI FR GB GR
   IE IT LI LU MC NL PT SE SK TR**
   Benannte Erstreckungsstaaten:
   **AL LT LV MK RO SI**

(30) Priorität: **07.09.2001   DE 10143995**

(71) Anmelder: **Pulsion Medical Systems AG
   81829 München (DE)**

(72) Erfinder:
   • **Pfeiffer, Ulrich J., Dr.Dr.
      81667 München (DE)**
   • **Burger, Thorsten, Dr.
      80804 München (DE)**

(74) Vertreter: **Kehl, Günther, Dipl.-Phys.
   Patentanwaltskanzlei
   Günther Kehl
   Friedrich-Herschel-Strasse 9
   81679 München (DE)**

(54) **System und Computerprogramm zur Bestimmung von Kreislaufgrössen eines Patienten**

(57)   Ein System zur Bestimmung von Kreislaufgrößen eines Patienten weist eine Vorrichtung zur nichtinvasiven Messung des qualitativen zeitlichen Verlaufs der lokalen Konzentration eines an einer ersten Stelle in den Blutkreislauf injizierten Indikators an einer zweiten Stelle des Blutkreislaufs auf. Ferner ist eine Auswerteeinheit vorgesehen, welche eine Eingangsschnittstelle zum Einlesen eines Eingangswerts des Herzzeitvolumens COe des Patienten aufweist, und in welcher ein Auswertealgorithmus implementiert ist, der den qualitativen zeitlichen Verlauf der lokalen Konzentration des in den Blutkreislauf injizierten Indikators in einen quantitativen zeitlichen Verlauf der lokalen Konzentration des in den Blutkreislauf injizierten Indikators transformiert. Bei der Transformation ist die Bedingung erfüllt ist, daß das aus dem quantitativen zeitlichen Verlauf der lokalen Konzentration gemäß einer vorgegebenen Beziehung berechenbare Herzzeitvolumen COdye gleich dem Eingangswert des Herzzeitvolumens COe ist. Mit dem System kann eine Kalibrierung der Indikatorkonzentrationsmessung erfolgen. Mit dem so gewonnenen quantitativen Indikatorkonzentrationsverlauf kann unter anderem das zirkulierende Blutvolumen bestimmt werden.

Fig. 1

EP 1 290 976 A2

## Beschreibung

Die vorliegende Erfindung betrifft ein System sowie ein Computerprogramm zur Bestimmung von Kreislaufgrößen eines Patienten, insbesondere zur Bestimmung des zirkulierenden Blutvolumens sowie hiervon abgeleiteter Größen mittels pulsspektrophotometrischer Methoden.

Ein zur Bestimmung des zirkulierenden Blutvolumens geeignetes System ist unter anderem aus der Offenlegungsschrift DE 41 30 931 A1 bekannt. Es basiert auf dem Prinzip der Pulsspektrophotometrie. Nach Injektion eines Farbstoff-Bolus in den Blutkreislauf eines Patienten wird der Farbstoffkonzentrationszeitverlauf optisch gemessen. Dies kann invasiv mittels eines faseroptischen Katheters oder nichtinvasiv mittels einer Reflexions- oder Transmissionsmessung an Finger, Ohrläppchen etc. erfolgen. Es wird eine Kreislauftransportfunktion bestimmt und aus dieser eine Kreislaufimpulsantwort berechnet, die einer idealen Indikatorinjektion zu einem Zeitpunkt t=0 entsprechen würde. Durch Rückextrapolation der Kreislaufimpulsantwort wird eine virtuelle Farbstoffkonzentration zum Zeitpunkt t=0 berechnet. Das zirkulierende Blutvolumen ergibt sich als Quotient aus der injizierten Farbstoffmenge und der virtuellen Farbstoffkonzentration zum Zeitpunkt t=0. Bei invasiver Messung kommt ein faseroptischer Katheter zum Einsatz, der technisch relativ aufwendig und teuer ist und dessen Applizierung zudem eine Belastung des Patienten darstellt. Bei nichtinvasiver Messung ergibt sich ein Problem mangelnder Meßgenauigkeit auf welches im folgenden eingegangen wird.

Für die Bestimmung einer Vielzahl von Kreislaufgrößen, u.a. des zirkulierenden Blutvolumens und des Herzzeitvolumens CO mittels pulsspektrophotometrischer Methoden ist die möglichst genaue Kenntnis des Verlaufs der Absolutkonzentration des in den Kreislauf injizierten Farbstoffs erforderlich, ein qualitativer Farbstoffkonzentrationszeitverlauf ist nicht ausreichend. Da das optische Verhalten von biologischem Gewebe wesentlich durch Lichtstreuung determiniert wird und das Lambert-Beersche Gesetz somit nicht anwendbar ist, können durch nichtinvasive spektrophotometrische Messung allerdings keine Absolutkonzentrationen ermittelt werden. Ein Ansatz zur Lösung diese Problems ist aus EP 0 505 918 bekannt, indem das Verhältnis der Konzentration des injizierten Farbstoffs zur Konzentration des in einem unterschiedlichen Wellenlängenbereich maximal absorbierenden, im Blut ohnehin gleichmäßig vorhandenen Hämoglobins als Referenzfarbstoff bestimmt wird. Hierzu wird die Extinktion bei den jeweiligen Absorptionsmaxima durch Messung mit zwei unterschiedlichen Wellenlängen bestimmt. Die Absolutkonzentration des Hämoglobins wird nach Blutentnahme in vitro gemessen. Somit erfolgt eine Kalibrierung, die allerdings aufgrund der In-Vitro-Messung keine Online-Kalibrierung darstellt.

Andere herkömmliche nichtinvasive pulsspektrophotometrische Systeme, wie das in US 5,999,841 offenbarte System zur Messung des zirkulierenden Blutvolumens, verzichten auf eine Kalibrierung mittels Blutentnahme und In-Vitro-Messung der Hämoglobin-Konzentration. Die im wesentlichen auf dem Lambert-Beerschen Gesetz beruhenden Auswertungsalgorithmen können allerdings aufgrund der oben genannten Streuungseinflüsse biologischen Gewebes nicht immer eine hinreichende Genauigkeit der ermittelten Farbstoffkonzentrationszeitverläufe und der hieraus bestimmten Kreislaufgrößen gewährleisten.

Der vorliegenden Erfindung liegt angesichts der geschilderten Probleme bei herkömmlichen Systemen der eingangs beschriebenen Art die Aufgabe zugrunde, ein System zu schaffen, welches eine höhere Genauigkeit und Zuverlässigkeit bei der Bestimmung von Kreislaufgrößen gestattet, welche bei der Ermittlung mittels Indikatorkonzentrationsmeßmethoden die Kenntnis der absoluten Indikatorkonzentration erfordern. Unter einem Indikator ist in diesem Zusammenhang ein nachweisbarer Stoff zu verstehen, der während eines relevanten Meßzeitraums im Gefäßsystem verbleibt. Insbesondere können als Indikatoren Stoffe verwendet werden, die im sichtbaren oder unsichtbaren Spektralbereich elektromagnetische Strahlung absorbieren. Dabei kommen vor allem Farbstoffe in Frage. Insbesondere liegt der Erfindung die Aufgabe zugrunde, ein System zu schaffen, mit dessen Hilfe sich das zirkulierende Blutvolumen und hiervon abgeleitete Größen mit höherer Genauigkeit und Zuverlässigkeit bestimmen lassen, als mit herkömmlichen auf dem Prinzip der Pulsspektrophotometrie basierenden Systemen.

Nach einem Aspekt der Erfindung wird diese Aufgabe durch ein System gemäß dem unabhängigen Anspruch 1 gelöst.

Bevorzugte Ausführungsformen des erfindungsgemäßen Systems sind in den Ansprüchen 2 bis 29 beschrieben.

Das System sieht also gleichsam eine Kalibrierung der Indikatorkonzentrationsmessung mittels eines anderweitig bestimmten Eingangswerts des Herzzeitvolumens COe vor, der vorzugsweise aus einer (vorzugsweise transpulmonalen) Thermodilutionsmessung stammt. Aus dem so gewonnenen quantitativen Indikatorkonzentrationsverlauf läßt sich durch Rückextrapolation des exponentiell abfallenden Kurvenasts auf die Ordinate die auf das zirkulierende Blutvolumen TBV bezogene Anfangskonzentration des injizierten Indikators ermitteln. Durch Bildung des Quotienten aus der injizierten Indikatormenge und der ermittelten auf das zirkulierende Blutvolumen TBV bezogenen Anfangskonzentration kann dann das zirkulierende Blutvolumen TBV berechnet werden. Gegenüber den oben beschriebenen auf Farbstoffkonzentrationsmessung beruhenden Systemen, bei welchen die In-Vitro-Bestimmung der Hämoglobin-Konzentration als Referenzfarbstoff zum Erhalt absoluter Konzentrationswerte erforderlich ist, ist also erfindungsgemäß vorzugsweise eine Kalibrierung mittels zeitgleich und online ermittelter Werte vorgesehen.

Wird eine transpulmonale Thermodilutionsmessung vorgesehen, was den Vorteil hat, daß kein wegen des hohen Anwendungsrisikos problematischer Swan-Ganz-Katheter verwendet zu werden braucht, so eignet sich als erste Stelle zur Injektion des den Indikator enthaltenden gekühlten Bolus beispielsweise die obere Hohlvene, zur Messung des zeitlichen Verlaufs der lokalen Bluttemperatur die Arteria femoralis oder die Arteria axillaris.

Durch die Kombination der Indikatorkonzentrationsmessung mit einer transpulmonalen Thermodilutionsmessung lassen sich zudem Kreislaufgrößen bestimmen, die einer reinen Indikatorkonzentrationsmessung aus prinzipiellen Gründen nicht zugänglich sind, insbesondere das intrathorakale Thermovolumen ITTV und das hieraus berechenbare extravasale Thermovolumen ETV, welches eng mit dem extravasalen Lungenwasser korreliert, sofern kein signifikanter Perfusionsdeffekt in den Lungen, etwa eine Lungenembolie, vorliegt.

Besonders vorteilhaft für die Bestimmung des qualitativen zeitlichen Verlaufs der lokalen Konzentration des vorzugsweise als Indikator eingesetzten Farbstoffs Indocyaningrüns ist eine Wellenlänge von 805 nm deshalb, weil bei 805 nm das Absorptionsmaximum von Indocyaningrün liegt.

Nach einem weiteren Aspekt der Erfindung wird ein Computerprogramm zur Bestimmung von Kreislaufgrößen eines Patienten gemäß dem unabhängigen Anspruch 30 bereitgestellt.

Bevorzugte Ausführungsformen des erfindungsgemäßen Computerprogramms sind in den Ansprüchen 31 bis 51 beschrieben.

Gemäß einem weiteren Aspekt der vorliegenden Erfindung wird ein Speichermedium gemäß Anspruch 52 bereitgestellt.

Nachfolgend wird eine bevorzugte Ausführungsform der Erfindung anhand der zugehörigen Zeichnungen als Beispiel näher erläutert.

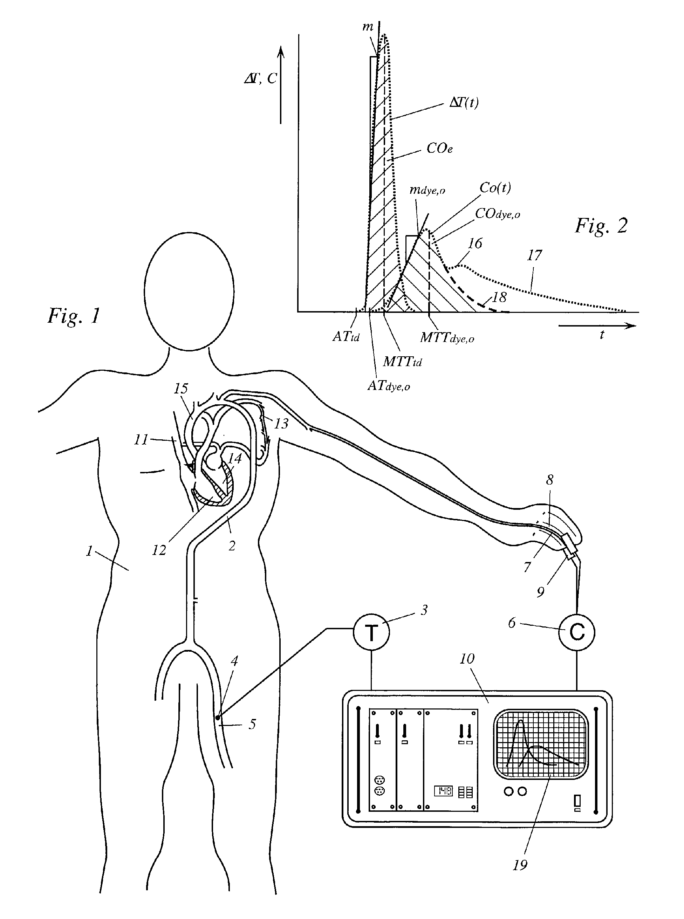

Fig. 1    zeigt hierbei rein schematisch den Aufbau eines erfindungsgemäßen Systems sowie einen Ausschnitt des Blutkreislaufsystems eines Patienten, dessen Kreislaufgrößen zu bestimmen sind, bei der Anwendung des Systems.

Fig. 2    zeigt ebenfalls rein schematisch den mit dem erfindungsgemäßen System nach Injektion eines unter die Körpertemperatur gekühlten, Indikator enthaltenden Bolus in den Blutkreislauf ermittelten zeitlichen Verlauf der Abweichung der lokalen Blutemperatur $\Delta T(t)$ gegenüber der Normaltemperatur sowie den entsprechenden qualitativen zeitlichen Verlauf der lokalen Konzentration des in den Blutkreislauf injizierten Indikators vor der Transformation in einen korrigierten quantitativen zeitlichen Verlauf der lokalen Konzentration $C_o(t)$ des in den Blutkreislauf injizierten Indikators. Die Abszisse fungiert als Zeitachse, die Ordinate als Temperatur- bzw. Konzentrationsachse.

Fig. 1 zeigt den schematischen Aufbau eines erfindungsgemäßen Systems. Beschrieben wird das System in seiner Anwendung zur Bestimmung von Kreislaufgrößen eines Patienten 1, dessen Blutkreislaufsystem 2 ausschnittweise und ebenfalls schematisch mit dargestellt ist. Das System weist einen Temperatursensor 3 auf, dessen vorgesehener Meßort 4 in der Arteria Femoralis 5 des Patienten 1 durch Einsatz eines arteriellen Katheters (nicht dargestellt) zugänglich ist. Weiter ist das System mit einer spektrometrischen Konzentrationsmeßeinheit 6 zur Messung der lokalen Konzentration an Indocyaningrün (ICG) in den Blutgefäßen 7 eines Fingers 8 des Patienten 1 ausgestattet. Hierzu weist die Konzentrationsmeßeinheit 6 zwei Lichtsender (nicht dargestellt) zum Aussenden von elektromagnetischen Wellen im nahinfraroten Bereich sowie zwei zugehörige NIR-Sensoren auf, die in einen Fingerclip 9 integriert sind. Die Lichtsender sind zur Erzeugung elektromagnetischer Strahlung mit einer Wellenlänge von 805 nm, was dem Absorptionsmaximum von ICG entspricht, bzw. etwa 900 nm ausgelegt. Die Nahinfrarotstrahlung wird mittels Lichtleitern, d.h. faseroptischen Bündeln (nicht dargestellt), zu Austrittsstellen am Fingerclip 9 geleitet, wo sie emittiert wird. Alternativ können auch direkt am Fingerclip 9 Leuchtdioden angebracht sein, die Strahlung der gewünschten Wellenlängen emittieren. Die den Austrittstellen gegenüber angeordneten NIR-Sensoren erfassen den jeweils durch den Finger transmittierten, nicht absorbierten Anteil der emittierten Strahlung. Auch eine Anordnung mit nur einem NIR-Sensor, der sowohl bei 805 nm als auch bei 900 nm ausreichende Empfindlichkeit besitzt, ist denkbar. In diesem Fall werden NIR-Lichtpulse von 805 nm bzw. 900 nm kurz nacheinander, d.h. zeitlich versetzt zueinander ausgesandt, so daß der Sensor die empfangene Strahlung aufgrund des Zeitpunkts jeweils der einen oder anderen Wellenlänge zuordnen kann.

Je geringer die Intensität der transmittierten Strahlung der Wellenlänge von 805 nm im Verhältnis zur Intensität der transmittierten Strahlung der Wellenlänge 900 nm ist, desto höher ist die Konzentration des im Blut enthaltenen Indocyaningrüns. Die Umrechnung der gemessenen Intensitäten in relative Konzentrationswerte erfolgt nach aus dem Gebiet der Pulsoxymetrie bekannten Verfahren.

Sowohl der Temperatursensor 3 als auch die spektrometrische Konzentrationsmeßeinheit 6 sind mit der in einen Rechner 10 integrierten Steuer- und Auswerteinheit verbunden. Auf dem Rechner 10 ist ein erfindungsgemäßes Computerprogramm installiert, welches den unten in seiner Funktion beschriebenen Auswertealgorithmus enthält.

Anhand des im folgenden beschriebenen Ablaufs einer typischen Messung wird die Funktionsweise des erfindungsgemäßen Systems und des auf dem Rechner 10 installierten erfindungsgemäßen Programms besonders deutlich.

Eine definierte Menge unter Körpertemperatur gekühlten Indocyaningrüns wird als Bolus in die obere Hohlvene 11 des Patienten 1 injiziert. Die Indikatormoleküle sowie die lokale Temperaturabweichung werden mit dem Blutstrom durch die rechte Herzkammer 12, den Lungenkreislauf 13 und die linke Herzkammer 14 des Patienten 1 transportiert und gelangen in die Aorta 15. Der weiter stromabwärts an den Meßorten in der Arteria Femoralis 5 und den Gefäßen 7 des Fingers 9 gemessene lokale Temperaturverlauf $\Delta T(t)$ bzw. qualitative Indikatorkonzentrationsverlauf Co(t) werden als Systemantwort des Kreislaufsystems auf die Bolusinjektion registriert und dienen dem Auswertealgorithmus des auf dem Rechner 10 installierten Computerprogramms als Eingangsdaten.

Ein jeweils typischer Temperaturverlauf $\Delta T(t)$ - auch als Thermodilutionskurve bezeichnet - und qualitativer Indikatorkonzentrationsverlauf Co(t) sind in Fig. 2 dargestellt. Der lokale zeitliche Temperaturunterschied $\Delta T$ ist positiv aufgetragen, wobei zu beachten ist, daß de facto eine lokale zeitliche Abkühlung vorliegt. Aufgrund der längeren Transportzeit bis zum Meßort der Konzentration, erkennbar an der gegenüber der Erscheinungszeit der Temperaturänderung ATtd späteren nicht korrigierten Erscheinungszeit der Konzentrationsänderung ATdye,o, fällt der qualitative Indikatorkonzentrationsverlauf Co(t) wegen diffusiver Ausbreitung der Indikatormoleküle flacher und breiter aus als der Temperaturverlauf $\Delta T(t)$. Dies ist erkennbar anhand der eingetragenen Steigungsdreiecke m bzw. mdye,o von Approximationsgeraden, die an die jeweilige ansteigende Flanke der Verläufe angelegt sind. Die Steigung m bzw. mdye,o entspricht hierbei jeweils der mittleren Steigung der ansteigenden Flanke in einem Bereich von 20% bis 80% des Maximums des jeweiligen Verlaufs. Zudem ist ein zweiter Konzentrationsanstieg 16 aufgrund von Rezirkulation von Indikatormolekülen erkennbar, bevor ein exponentieller Abfall 17 der Indikatorkonzentration auftritt, der vom Abbau des Indocyaningrün in der Leber herrührt. Für den Bereich des exponentiellen Abfalls 17 wird von einer gleichmäßigen Indikatorkonzentration im Gefäßsystem ausgegangen.

Ferner sind eingetragen die mittlere Transitzeit der Temperaturänderung MTTtd sowie die nicht korrigierte mittlere Transitzeit des Indikators MTTdye,o. Für das Zustandekommen des Temperaturverlaufs $\Delta T(t)$ ist zu bedenken, daß entlang des Transportwegs durch den Lungenkreislauf 13 ein Wärmeaustausch beispielsweise mit extravasalem Lungenwasser stattfindet, woraus eine Verschiebung der mittleren Transitzeit der Temperaturänderung MTTtd resultiert.

Der Auswertealgorithmus transformiert den qualitativen Indikatorkonzentrationsverlauf Co(t) in einen korrigierten quantitativen Indikatorkonzentrationsverlauf (nicht dargestellt). Die Transformation wird so durchgeführt, daß die folgenden drei Bedingungen erfüllt sind:

1) Das sich aus dem korrigierten quantitativen zeitlichen Verlauf der lokalen Konzentration ergebende Herzzeitvolumen COdye ist gleich dem sich aus dem zeitlichen Verlauf der lokalen Temperaturänderung $\Delta T(t)$ ergebenden Herzzeitvolumen COe. Hierbei wird auf die bekannte Stewart-Hamilton-Beziehung für das Herzzeitvolumen CO zurückgegriffen, was in der Hervorhebung der Fläche unter der Thermodilutionskurve versinnbildlicht ist.

Zu beachten ist, daß für die Ermittlung von COdye rezirkulierter Indikator unberücksichtigt bleiben muß. Dies kann etwa durch Extrapolation des ersten abfallenden Kurvenasts des Indikatorkonzentrationszeitverlaufs auf die Abszisse erfolgen. Aus Illustrationsgründen ist eine solche Extrapolation 18 für den qualitativen Indikatorkonzentrationszeitverlauf dargestellt.

Günstiger ist es allerdings, für die Ermittlung von COdye nur auf den ansteigenden Kurvenast des Indikatorkonzentrationszeitverlaufs bis zum Maximum zurückzugreifen, und sich einer Beziehung zu bedienen, die für intravasal gemessene Dilutionskurven bekannt ist: Demnach entspricht die Fläche unter einer typischen intravasal z.B. in der Arteria femoralis oder Arteria axillaris gemessenen Dilutionskurve etwa dem 2,5- bis 3-fachen, in der Regel dem 2,58-fachen, der Fläche unter dem ansteigenden Kurvenast des Indikatorkonzentrationszeitverlaufs bis zum Maximum. Dies hat sich aus der Auswertung einer Vielzahl intravasal gemessener Dilutionskurven bestätigt. Zweckmäßigerweise ermittelt man daher COdye als einen Wert, der dem Kehrwert des Integrals des Indikatorkonzentrationszeitverlaufs über die Zeit bis zum Maximum proportional ist. Um den Rechenaufwand zu vereinfachen, kann hierzu der ansteigende Kurvenast auch linear approximiert werden.

2) Ein definierter Bereich, beispielsweise von 20% bis 80% des Maximums, der ansteigenden Flanken des quantitativen korrigierten zeitlichen Verlaufs der lokalen Konzentration sowie des zeitlichen Verlaufs der lokalen Bluttemperatur $\Delta T(t)$ weist jeweils die gleiche mittlere Steigung m auf.

Ersatzweise ist die Bedingung so zu formulieren, daß ein definierter Bereich, beispielsweise von 20% bis 80% des Maximums, des ansteigenden Kurvenasts sich über eine Zeit erstreckt, die in vorgegebener Relation zu einem aus intravasaler Messung gewonnenen Vergleichswert steht. Auch hier kann auf eine lineare Approximation des ansteigenden Kurvenastes zurückgegriffen werden. Die Erfüllung dieser Bedingung läuft letztlich auf eine, in guter Näherung lineare, Transformation der Abszisse hinaus.

3) Die Erscheinungszeit ATdye des quantitativen korrigierten zeitlichen Verlaufs der lokalen Indikatorkonzentration ist gleich der Erscheinungszeit ATtd des zeitlichen Verlaufs der lokalen Bluttemperatur $\Delta T(t)$.

Aus dem so gewonnenen korrigierten quantitativen Indikatorkonzentrationsverlauf ermittelt der Auswertealgorithmus durch Rückextrapolation des exponentiell abfallenden Kurvenasts auf die Ordinate zum Zeitpunkt der Injektion oder näherungsweise zum Erscheinungszeitpunkt ATdye die auf das zirkulierende Blutvolumen TBV bezogene Anfangskonzentration des injizierten Indikators. Durch Bildung des Quotienten aus der injizierten Indikatormenge und der ermittelten auf das zirkulierende Blutvolumen TBV bezogenen Anfangskonzentration wird das zirkulierende Blutvolumen TBV berechnet.

Ferner umfaßt der Auswertealgorithmus die Berechnung der folgenden Kreislaufgrößen: das intrathorakale Blutvolumen ITBV als Produkt des Herzzeitvolumens CO und der aus dem korrigierten quantitativen zeitlichen Verlauf der lokalen Konzentration des in den Blutkreislauf injizierten Indikators gewonnenen mittleren Transitzeit MTTdye, ferner das intrathorakale Thermovolumen ITTV als Produkt des Herzzeitvolumens COe und der aus dem zeitlichen Verlauf der lokalen Bluttemperatur gewonnenen mittleren Transitzeit MTTtd.

Mittels Bildung der Differenz aus intrathorakalem Blutvolumen ITBV und intrathorakalem Thermovolumen ITTV berechnet der Auswertealgorithmus das extravasale Thermovolumen ETV, welches mit dem extravasalen Lungenwasser EVLW korreliert.

Die perifere Perfusion wird mittels Bildung der Differenz aus der aus dem korrigierten quantitativen zeitlichen Verlauf der lokalen Konzentration des in den Blutkreislauf injizierten Indikators gewonnenen mittleren Transitzeit MTTdye und der aus dem qualitativen zeitlichen Verlauf der lokalen Konzentration des in den Blutkreislauf injizierten Indikators gewonnenen mittleren Transitzeit MTTdye,o berechnet.

Ferner berechnet der Auswertealgorithmus die Abbaurate des Indikators, die PDR, aus dem zeitlich exponentiellen Abfall des quantitativen oder des qualitativen zeitlichen Verlaufs der lokalen Konzentration des in den Blutkreislauf injizierten Indikators nach angenommener vollständiger Vermischung.

Die ermittelten Kreislaufgrößen werden auf einem Monitor 19 angezeigt und zudem auf einem Datenträger, etwa einer Diskette oder einem Speichchip, gespeichert. Über eine geeignete Schnittstelle können die ermittelten Kreislaufgrößen auch mittels eines Druckers zu Katalogisierungs- oder Dokumentationszwecken ausgegeben werden.

**Patentansprüche**

1. System zur Bestimmung von Kreislaufgrößen eines Patienten, aufweisend eine Vorrichtung zur nichtinvasiven Messung des qualitativen zeitlichen Verlaufs der lokalen Konzentration eines an einer ersten Stelle in den Blutkreislauf injizierten Indikators an einer zweiten Stelle des Blutkreislaufs, sowie eine Auswerteeinheit,
   welche eine Eingangsschnittstelle zum Einlesen eines Eingangswerts des Herzzeitvolumens COe des Patienten aufweist,
   und in welcher ein Auswertealgorithmus implementiert ist, der den qualitativen zeitlichen Verlauf der lokalen Konzentration des in den Blutkreislauf injizierten Indikators in einen quantitativen zeitlichen Verlauf der lokalen Konzentration des in den Blutkreislauf injizierten Indikators transformiert,
   wobei die Bedingung erfüllt ist, daß das aus dem quantitativen zeitlichen Verlauf der lokalen Konzentration gemäß einer vorgegebenen Beziehung berechenbare Herzzeitvolumen COdye gleich dem Eingangswert des Herzzeitvolumens COe ist.

2. System nach Anspruch 1, wobei das Herzzeitvolumen COdye gemäß der vorgegebenen Beziehung proportional dem Kehrwert des Integrals des quantitativen zeitlichen Verlaufs der lokalen Konzentration über die Zeit bis zum Maximum des quantitativen zeitlichen Verlaufs der lokalen Konzentration ist.

3. System nach Anspruch 1, wobei das Herzzeitvolumen COdye gemäß der vorgegebenen Beziehung proportional dem Kehrwert des Integrals einer linearen Näherung der ansteigenden Flanke des quantitativen zeitlichen Verlaufs der lokalen Konzentration über die Zeit bis zum Maximum des quantitativen zeitlichen Verlaufs der lokalen Konzentration ist.

4. System nach einem der Ansprüche 1-3, wobei die Eingangsschnittstelle mit einer Vorrichtung zur Messung des zeitlichen Verlaufs der lokalen Bluttemperatur an einer dritten Stelle des Blutkreislaufs des Patienten nach Injektion eines unter die Körpertemperatur gekühlten, den Indikator enthaltenden Bolus in den Blutkreislauf an der ersten Stelle verbunden ist,
   welche dazu eingerichtet ist, aus dem zeitlichen Verlauf der lokalen Bluttemperatur nach gängigen Methoden der Thermodilutionsmessung den Eingangswert des Herzzeitvolumens COe zu bestimmen und auszugeben.

5. System nach einem der Ansprüche 2-4, wobei der in die Auswerteeinheit implementierte Auswertealgorithmus den qualitativen zeitlichen Verlauf der lokalen Konzentration des in den Blutkreislauf injizierten Indikators in einen korrigierten quantitativen zeitlichen Verlauf der lokalen Konzentration des in den Blutkreislauf injizierten Indikators transformiert, indem die Zeitachse so transformiert wird, daß ein definierter Bereich der ansteigenden Flanke des quantitativen korrigierten zeitlichen Verlaufs der lokalen Konzentration sich über eine Zeit erstreckt, die in vorgegebener Relation zu einem Vergleichswert steht.

6.  System nach Anspruch 5, wobei die Zeitachse mittels einer Transportfunktion g(t) transformiert wird.

7.  System nach den Ansprüchen 4, 5 und 6, wobei die Transportfunktion g(t) mithilfe der Gleichung

$$Co*(t) = \int_{ATtd}^{T\max} g(t-u)\Delta T(u)du$$

approximiert wird, wobei Co*(t) die ansteigende Flanke der qualitativen Indikatorkonzentrationskurve, ATtd der Erscheinungszeitpunkt des gekühlten Bolus, $\Delta T(t)$ der zeitliche Verlauf der lokalen Bluttemperaturänderung, Tmax der Zeitpunkt, bei dem die lokale Bluttemperaturänderung ihren Maximalwert erreicht, und u eine Integrationsvariable ist.

8.  System nach Anspruch 5, wobei die Zeitachse linear transformiert wird.

9.  System nach Anspruch 5, wobei der Vergleichswert der Zeitdauer entspricht, über welche sich ein, dem definierten Bereich der ansteigenden Flanke des quantitativen korrigierten zeitlichen Verlaufs entsprechender, definierter Bereich der ansteigenden Flanke des zeitlichen Verlaufs der lokalen Bluttemperatur erstreckt.

10.  System nach einem der Ansprüche 2-4, wobei der in die Auswerteeinheit implementierte Auswertealgorithmus den qualitativen zeitlichen Verlauf der lokalen Konzentration des in den Blutkreislauf injizierten Indikators in einen korrigierten quantitativen zeitlichen Verlauf der lokalen Konzentration des in den Blutkreislauf injizierten Indikators transformiert, indem die Zeitachse so transformiert wird, daß ein definierter Bereich der ansteigenden Flanke des quantitativen korrigierten zeitlichen Verlaufs der lokalen Konzentration eine mittlere Steigung m aufweist, die in vorgegebener Relation zu einem Vergleichswert steht.

11.  System nach Anspruch 10, wobei die Zeitachse linear transformiert wird.

12.  System nach Anspruch 10, wobei der Vergleichswert der mittleren Steigung entspricht, über welche sich ein, dem definierten Bereich der ansteigenden Flanke des quantitativen korrigierten zeitlichen Verlaufs entsprechender, definierter Bereich der ansteigenden Flanke des zeitlichen Verlaufs der lokalen Bluttemperatur erstreckt.

13.  System nach einem der Ansprüche 5-12, wobei der definierte Bereich sich von 20% bis 80% des Maximums des quantitativen korrigierten zeitlichen Verlaufs der lokalen Konzentration erstreckt.

14.  System nach Anspruch 12, wobei bei der Transformation zusätzlich die Bedingung erfüllt ist, daß der quantitative korrigierte zeitliche Verlauf der lokalen Konzentration die gleiche Erscheinungszeit AT aufweist wie der zeitliche Verlauf der lokalen Bluttemperatur.

15.  System nach einem der vorangehenden Ansprüche, wobei der Auswertealgorithmus aus dem quantitativen zeitlichen Verlauf der lokalen Konzentration des in den Blutkreislauf injizierten Indikators die auf das zirkulierende Blutvolumen TBV bezogene Anfangskonzentration des injizierten Indikators ermittelt und durch Bildung des Quotienten aus der injizierten Indikatormenge und der ermittelten auf das zirkulierende Blutvolumen TBV bezogenen Anfangskonzentration das zirkulierende Blutvolumen TBV berechnet.

16.  System nach Anspruch 15, wobei die Anfangskonzentration des injizierten Indikators durch Rückextrapolation des zeitlichen Verlaufs der lokalen Konzentration des in den Blutkreislaufs injizierten Indikators bis zum Erscheinungszeitpunkt ATdye des Indikators oder bis zum Zeitpunkt der Injektion des Indikators ermittelt wird.

17.  System nach Anspruch 15, wobei der Auswertealgorithmus ferner das intrathorakale Blutvolumen ITBV als Produkt des Herzzeitvolumens COe und der aus dem korrigierten quantitativen zeitlichen Verlauf der lokalen Konzentration des in den Blutkreislauf injizierten Indikators gewonnenen mittleren Transitzeit MTT dye berechnet.

18.  System nach einem der Ansprüche 4-17, wobei der Auswertealgorithmus ferner das intrathorakale Thermovolumen ITTV als Produkt des Herzzeitvolumens COe und der aus dem zeitlichen Verlauf der lokalen Bluttemperatur gewonnenen mittleren Transitzeit MTTtd berechnet.

19.  System nach Anspruch 18, wobei der Auswertealgorithmus das extravasale Thermovolumen ETV mittels Bildung der Differenz aus intrathorakalem Blutvolumen ITBV und intrathorakalem Thermovolumen ITTV berechnet.

20. System nach einem der Ansprüche 14-19, wobei der Auswertealgorithmus die perifere Perfusion mittels Bildung der Differenz aus der aus dem korrigierten quantitativen zeitlichen Verlauf der lokalen Konzentration des in den Blutkreislauf injizierten Indikators gewonnenen mittleren Transitzeit MTTdye und der aus dem qualitativen zeitlichen Verlauf der lokalen Konzentration des in den Blutkreislauf injizierten Indikators gewonnenen mittleren Transitzeit MTTdye,o berechnet.

21. System nach einem der Ansprüche 14-19, wobei der Auswertealgorithmus die perifere Perfusion mittels Bildung der Differenz aus der aus dem korrigierten quantitativen zeitlichen Verlauf der lokalen Konzentration des in den Blutkreislauf injizierten Indikators gewonnenen Erscheinungszeit ATdye und der aus dem qualitativen zeitlichen Verlauf der lokalen Konzentration des in den Blutkreislauf injizierten Indikators gewonnenen Erscheinungszeit ATdye,o berechnet.

22. System nach einem der vorangehenden Ansprüche, wobei der Auswertealgorithmus ferner die Abbaurate des Indikators PDR aus dem zeitlich exponentiellen Abfall des quantitativen zeitlichen Verlaufs der lokalen Konzentration des in den Blutkreislauf injizierten Indikators nach angenommener vollständiger Vermischung berechnet.

23. System nach einem der vorangehenden Ansprüche, wobei der Auswertealgorithmus ferner den nach einer festgelegten Zeitspanne im Blut verbliebenen Indikatoranteil ermittelt.

24. System nach einem der vorangehenden Ansprüche, wobei der Indikator Indocyaningrün ist, und die Vorrichtung zur nichtinvasive Messung des qualitativen zeitlichen Verlaufs der lokalen Konzentration Mittel zur Aussendung nahinfraroter elektromagnetischer Wellen und einen im nahinfraroten Bereich empfindlichen Sensor aufweist.

25. System nach Anspruch 24, wobei es sich bei der nichtinvasiven Messung des qualitativen zeitlichen Verlaufs der lokalen Konzentration um eine an einem Finger, einer Zehe, der Nase oder einem Ohr des Patienten vorzunehmende Transmissionsmessung handelt.

26. System nach einem der Ansprüche 24-25, wobei die Mittel zur Aussendung nahinfraroter elektromagnetischer Wellen für die Erzeugung mindestens zweier unterschiedlicher Wellenlängen eingerichtet sind, und das System so ausgerüstet ist, daß Intensitäten der unterschiedlichen Wellenlängen mittels des Sensors jeweils selektiv erfaßt werden können.

27. System nach Anspruch 26, wobei eine der unterschiedlichen Wellenlängen etwa 805 nm beträgt.

28. System nach einem der Ansprüche 4-27, wobei in die Auswerteeinheit Mittel zur Bestimmung des Eingangswertes des Herzzeitvolumens COe aus dem zeitlichen Verlauf der lokalen Bluttemperatur nach gängigen Methoden der Thermodilutionsmessung integriert sind und die Eingangsschnittstelle zum Einlesen des Eingangswerts des Herzzeitvolumens COe eine virtuell oder physikalisch realisierte interne Schnittstelle der Auswerteeinheit ist.

29. System nach einem der vorangehenden Ansprüche, wobei das System ferner Mittel zur Messung des zeitlichen Verlaufs des zentralvenösen Blutdrucks sowie des zeitlichen Verlaufs des arteriellen Blutdrucks aufweist, und in die Auswerteeinheit ein weiterer Auswertealgorithmus zur Durchführung einer Pulskonturanalyse unter Verwendung der gemessenen zeitlichen Verläufe des zentralvenösen Blutdrucks sowie des arteriellen Blutdrucks implementiert ist.

30. Computerprogramm zur Bestimmung von Kreislaufgrößen eines Patienten, aufweisend einen von einem Rechner ausführbaren Auswertealgorithmus, der als Eingangsdaten den durch nichtinvasive Messung an einer zweiten Stelle des Blutkreislaufs ermittelten qualitativen zeitlichen Verlauf der lokalen Konzentration eines an einer ersten Stelle in den Blutkreislauf injizierten Indikators verarbeitet,
indem der Auswertealgorithmus den qualitativen zeitlichen Verlauf der lokalen Konzentration des in den Blutkreislauf injizierten Indikators in einen quantitativen zeitlichen Verlauf der lokalen Konzentration des in den Blutkreislauf injizierten Indikators transformiert,
wobei die Bedingung erfüllt ist, daß das aus dem quantitativen zeitlichen Verlauf der lokalen Konzentration gemäß einer vorgegebenen Beziehung berechenbare Herzzeitvolumen COdye gleich einem anderweitig bestimmten Eingangswert des Herzzeitvolumens COe ist.

31. Computerprogramm nach Anspruch 30, wobei das Herzzeitvolumen COdye gemäß der vorgegebenen Beziehung proportional dem Kehrwert des Integrals des quantitativen zeitlichen Verlaufs der lokalen Konzentration über die Zeit bis zum Maximum des quantitativen zeitlichen Verlaufs der lokalen Konzentration ist.

32. Computerprogramm nach Anspruch 30, wobei das Herzzeitvolumen COdye gemäß der vorgegebenen Beziehung proportional dem Kehrwert des Integrals einer linearen Näherung der ansteigenden Flanke des quantitativen zeitlichen Verlaufs der lokalen Konzentration über die Zeit bis zum Maximum des quantitativen zeitlichen Verlaufs der lokalen Konzentration ist.

**33.** Computerprogramm gemäß einem der Ansprüche 30-32, wobei der Auswertealgorithmus als weitere Eingangsdaten den an einer dritten Stelle des Blutkreislaufs des Patienten gemessenen zeitlichen Verlauf der lokalen Bluttemperatur nach Injektion eines unter die Körpertemperatur gekühlten, den Indikator enthaltenden Bolus in den Blutkreislauf an der ersten Stelle verarbeitet, und den Eingangswert des Herzzeitvolumens COe nach gängigen Methoden der Thermodilutionsmessung bestimmt.

**34.** Computerprogramm gemäß einem der Ansprüche 30-33, wobei der Auswertealgorithmus den qualitativen zeitlichen Verlauf der lokalen Konzentration des in den Blutkreislauf injizierten Indikators in einen korrigierten quantitativen zeitlichen Verlauf der lokalen Konzentration des in den Blutkreislauf injizierten Indikators transformiert, indem
die Zeitachse so transformiert wird, daß ein definierter Bereich der ansteigenden Flanke des quantitativen korrigierten zeitlichen Verlaufs der lokalen Konzentration sich über eine Zeit erstreckt, die in vorgegebener Relation zu einem Vergleichswert steht.

**35.** Computerprogramm nach Anspruch 34, wobei die Zeitachse mittels einer Transportfunktion g(t) transformiert wird.

**36.** Computerprogramm nach den Ansprüchen 33-35, wobei die Transportfunktion g(t) mithilfe der Gleichung

$$Co*(t) = \int_{ATtd}^{T\max} g(t-u)\Delta T(u)du$$

approximiert wird, wobei Co*(t) die ansteigende Flanke der qualitativen Indikatorkonzentrationskurve, ATtd der Erscheinungszeitpunkt des gekühlten Bolus, $\Delta T(t)$ der zeitliche Verlauf der lokalen Bluttemperaturänderung, Tmax der Zeitpunkt, bei dem die lokale Bluttemperaturänderung ihren Maximalwert erreicht, und u eine Integrationsvariable ist.

**37.** Computerprogramm nach Anspruch 34, wobei die Zeitachse linear transformiert wird.

**38.** Computerprogramm nach Anspruch 34, wobei der Vergleichswert der Zeitdauer entspricht, über welche sich ein, dem definierten Bereich der ansteigenden Flanke des quantitativen korrigierten zeitlichen Verlaufs entsprechender, definierter Bereich der ansteigenden Flanke des zeitlichen Verlaufs der lokalen Bluttemperatur erstreckt.

**39.** Computerprogramm gemäß einem der Ansprüche 30-33, wobei der Auswertealgorithmus den qualitativen zeitlichen Verlauf der lokalen Konzentration des in den Blutkreislauf injizierten Indikators in einen korrigierten quantitativen zeitlichen Verlauf der lokalen Konzentration des in den Blutkreislauf injizierten Indikators transformiert, indem
die Zeitachse so transformiert wird, daß ein definierter Bereich der ansteigenden Flanke des quantitativen korrigierten zeitlichen Verlaufs der lokalen Konzentration eine mittlere Steigung aufweist, die in vorgegebener Relation zu einem Vergleichswert steht.

**40.** Computerprogramm nach Anspruch 39, wobei die Zeitachse linear transformiert wird.

**41.** Computerprogramm nach Anspruch 39, wobei der Vergleichswert der mittleren Steigung entspricht, über welche sich ein, dem definierten Bereich der ansteigenden Flanke des quantitativen korrigierten zeitlichen Verlaufs entsprechender, definierter Bereich der ansteigenden Flanke des zeitlichen Verlaufs der lokalen Bluttemperatur erstreckt.

**42.** Computerprogramm gemäß einem der Ansprüche 34-41, wobei der definierte Bereich sich von 20% bis 80% des Maximums des quantitativen korrigierten zeitlichen Verlaufs der lokalen Konzentration erstreckt.

**43.** Computerprogramm nach Anspruch 39, wobei bei der Transformation zusätzlich die Bedingung erfüllt ist, daß der quantitative korrigierte zeitliche Verlauf der lokalen Konzentration die gleiche Erscheinungszeit AT aufweist wie der zeitliche Verlauf der lokalen Bluttemperatur.

**44.** Computerprogramm nach einem der Ansprüche 30-43, wobei der Auswertealgorithmus aus dem quantitativen zeitlichen Verlauf der lokalen Konzentration des in den Blutkreislauf injizierten Indikators die auf das zirkulierende Blutvolumen TBV bezogenen Anfangskonzentration des injizierten Indikators ermittelt und durch Bildung des Quotienten aus der injizierten Indikatormenge und der ermittelten auf das zirkulierende Blutvolumen TBV bezogenen Anfangskonzentration das zirkulierende Blutvolumen TBV berechnet.

**45.** Computerprogramm nach Anspruch 44, wobei die Anfangskonzentration des injizierten Indikators durch Rückextrapolation des zeitlichen Verlaufs der lokalen Konzentration des in den Blutkreislaufs injizierten Indikators bis zum Erscheinungszeitpunkt ATdye des Indikators oder bis zum Zeitpunkt der Injektion des Indikators ermittelt wird.

**46.** Computerprogramm nach Anspruch 44, wobei der Auswertealgorithmus ferner das intrathorakale Blutvolumen ITBV als Produkt des Herzzeitvolumens COe und der aus dem korrigierten quantitativen zeitlichen Verlauf der lokalen Konzentration des in den Blutkreislauf injizierten Indikators gewonnenen mittleren Transitzeit MTTdye berechnet.

**47.** Computerprogramm nach einem der Ansprüche 33-46, wobei der Auswertealgorithmus ferner das intrathorakale Thermovolumen ITTV als Produkt des Herzzeitvolumens COe und der aus dem zeitlichen Verlauf der lokalen Bluttemperatur gewonnenen mittleren Transitzeit MTTtd berechnet.

**48.** Computerprogramm nach Anspruch 47, wobei der Auswertealgorithmus das extravasle Thermovolumen ETV mittels Bildung der Differenz aus intrathorakalem Blutvolumen ITBV und intrathorakalem Thermovolumen ITTV berechnet.

**49.** Computerprogramm nach einem der Ansprüche 30-48, wobei der Auswertealgorithmus die perifere Perfusion mittels Bildung der Differenz aus der aus dem korrigierten quantitativen zeitlichen Verlauf der lokalen Konzentration des in den Blutkreislauf injizierten Indikators gewonnenen mittleren Transitzeit MTTdye und der aus dem qualitativen zeitlichen Verlauf der lokalen Konzentration des in den Blutkreislauf injizierten Indikators gewonnenen mittleren Transitzeit MTTdye,oberechnet.

**50.** Computerprogramm nach einem der Ansprüche 30-48, wobei der Auswertealgorithmus die perifere Perfusion mittels Bildung der Differenz aus der aus dem korrigierten quantitativen zeitlichen Verlauf der lokalen Konzentration des in den Blutkreislauf injizierten Indikators gewonnenen Erscheinungszeit ATdye und der aus dem qualitativen zeitlichen Verlauf der lokalen Konzentration des in den Blutkreislauf injizierten Indikators gewonnenen Erscheinungszeit ATdye,o berechnet.

**51.** Computerprogramm nach einem der Ansprüche 30-50, wobei der Auswertealgorithmus ferner die Abbaurate des Indikators PDR aus dem zeitlich exponentiellen Abfall des quantitativen oder qualitativen zeitlichen Verlaufs der lokalen Konzentration des in den Blutkreislauf injizierten Indikators nach angenommener vollständiger Vermischung berechnet.

**52.** Speichermedium, auf welchem physikalisch ein Computerprogramm nach einem der Ansprüche 30-51 gespeichert ist.

*Fig. 1*

*Fig. 2*